# EUROPÄISCHE PATENTSCHRIFT

(11) **EP 0 469 007 B1**
(45) Veröffentlichungstag und Bekanntmachung des Hinweises auf die Patenterteilung: **07.07.1993**
(21) Anmeldenummer: 90906116.0
(22) Anmeldetag: 14.04.1990
(51) Int. Cl.: A61K 7/06, A61K 7/48, A61K 7/04

(54) **MITTEL ZUR NAGEL-, HAUT- UND HAARPFLEGE**
PREPARATION FOR CARE OF NAILS, SKIN AND HAIR
COMPOSITION POUR LE SOIN DES ONGLES, DE LA PEAU ET DES CHEVEUX

(30) Priorität: 20.04.1989 CH 1498/89; 09.12.1989 DE 3940724
(43) Veröffentlichungstag der Anmeldung: 05.02.1992
(73) Patentinhaber: STüCKLER, Erwin, D-79809 Weilheim (DE)
(72) Erfinder: STüCKLER, Erwin, D-79809 Weilheim (DE)
(74) Vertreter: Hiebsch, Gerhard F., Dipl.-Ing.
(86) Internationale Anmeldenummer: DE9000287
(87) Internationale Veröffentlichungsnummer: WO9012560

(56) Entgegenhaltungen:
- EP-A- 0 289 639

## Beschreibung

Die Erfindung betrifft ein Mittel zur Nagel-, Haut- und Haarpflege und zur Bekämpfung des fortschreitenden Haarausfalles und zum Anregen neuen Haarwuchses von Humanhaar.

Es ist aus der Europäischen Patentanmeldung 0 289 639 ein Mittel zur Wiederbelebung und zum Anregen und Verstärken des Haarwuchses bekannt. Das Mittel enthält das Alkaloid Trigonellin bzw. Trigonellinsäure. Es wird auch gelehrt, dass dem Mittel Vitamin B6 zugegeben werden soll.

Aus der Europäischen Patentanmeldung ist jedoch nicht bekannt geworden, welche Mengen des oder der Wirkstoffe in welcher Zeitabfolge beispielsweise dem menschlichen Körper zugeführt weiden müssen, um zum angegebenen Ziele zu gelangen.

Dies ist insofern nachteilig, als dass geringe Gaben an Trigonellin nicht die erhoffte Wirkung zeigen und bei zu hohen Dosierungen neben der Kostenfolge unter Umständen auch mit an sich unerwünschten Nebenwirkungen zu rechnen ist. Es wurde nun überraschend gefunden, dass regelmässige Erfolge mit einem neuen trigonellinhaltigen Mittel erzielt werden können. Es wurde weiter überraschenderweise gefunden, dass das vorliegende Mittel auch-für die Pflege von Nägeln, Haut und Haaren wertvoll ist.

Erfindungsgemäss wird dies durch ein Mittel, enthaltend in der täglich darzureichenden Form
a) 100 bis 500 mg Trigonellin und
b) 1 bis 5 mg Vitamin B6 erreicht.

Unter Trigonellin wird die Verbindung 3-Carboxy-1-methylpyridiniumhydroxid verstanden, die auch unter dem Trivialnamen Coffearin bekannt ist.

Die Mengenangaben beziehen sich auf die chemisch reine Form der Verbindungen.

Trigonellin kann in synthetisierter Form angewendet werden. Eine besonders wertvolle und daher auch bevorzugte Form des Trigonellins kann aus dem Samen des Bockshornklee extrahiert werden. Derartige Extrakte sind an sich schon lange bekannt und stellen eine Mischung verschiedener extrahierbarer Bestandteile des pflanzlichen Körpers, insbesondere des Samens der Pflanze der Gattung Trigonella foenum graecum dar. Das Trigonellin;lässt sich beispielsweise durch einen der bekannten Extraktionsprozesse, wie Mazerisation oder Perkolation gewinnen.

Mit Vitamin B6 wird die definierte Mischung von Pyridoxin, Pyridoxal und Pyridoxamin umfasst. Das Vitamin B6 ist in der Literatur hinreichend beschrieben und kann auf sythetischem Wege, halbsynthetischem Wege oder aus Naturprodukten gewonnen werden.

Bevorzugt ist ein Mittel, wie oben beschrieben, enthaltend
a) 100 bis 400 mg Trigonellin und
b) 2 bis 4 mg Vitamin B6.

Zu den bevorzugten Mitteln gehören auch solche, enthaltend 10 bis 50 mg Nicotinsäure und/oder Nicotinsäureamid.

Ein weiteres bevorzugetes Mittel kann 1 bis 4 mg Riboflavin enthalten.

Weitere bevorzugte Mittel enthalten 0,2 bis 1mg Folsäure. Zu den Mitteln die auch bevorzugt werden sind diejenigen zu zählen, die 5 bis 25 mg d-Calcium-Pantothenat enthalten.

Besonders bevorzugt sind Mittel, die wenigstens zwei der Substanzen Nicotinsäure und/oder Nicotinsäureamid, Riboflavin, Folsäure und/oder d-Calcium-Pantothenat enthalten.

Die genannten Substanzen sind an sich und d-Calcium-Pantothenat auch aus der Haarkosmetik bekannt.

Es wurde gefunden, dass die oben angeführten bevorzugten Substanzen, die zur Vitamin B-Gruppe gehören, in den angegebenen Mengen, zusammen mit den erfindungsgemässen Mengen an Trigonellin und Vitamin B6 zu vorteilhaft wirkungsvollen Mitteln führen.

Die erfindungsgemässen Mittel können auch die Aminosäuren Cystein und/oder Lysin enthalten. Cystein und Lysin sind bekannte Verbindungen, wobei Lysin eine essentielle und Cystein eine nicht essentielle Aminosäure darstellt. Cystein an sich hat als für die Keratinbildung bekannter Stoff in der Haarkosmetik eingang gefunden.

Besonders bevorzugt sind Mittel, die beispielsweise Blütenpollen, enthaltend ihrerseits Riboflavin, Zystin und Rutin, enthalten. Die Menge an Blütenpollen kann beispielsweise 100 - 1500 Mg, zweckmässig 1000 - 1500 und vorzugsweise 1000 - 1300 mg betragen.

Auch bevorzugt ist ein Mittel, das einen oder mehrere Stoffe aus der Reihe von Calciumhydrogenphosphat, wässrigem Brennesselwurzelextrakt und Brennesselblätterextrakt, insbesondere von jungen Blättern, Thiaminnitrat (Vitamin B 1), Vitamin B 12, Vitamin B 8, Methionin, einer Mischung von Hystidin, Lysin und Arginin, vorzugsweise im Verhältnis 1:4:12, Knoblauchoel und/oder Knoblauchextrakt oder zumindest die darin enthaltenen Verbindungen Aliin und/oder Aliicin, einem Extrakt aus Brennessel, Huflattich, Schafgarbe, Rosmarin, Salbei, Schachtelhalm, Kleeblüte und/oder Birke, einem Komplex aus den Vitaminen A, E und/oder F, Vitamin H oder schwefelhaltigen Aminosäuren enthält.

Auch bevorzugt sind Mittel, wie oben beschrieben, die einen oder mehrere der nachfolgend genannten Stoffe enthalten, beispielsweise Bierhefe, wobei Mengen von z.B. 10 bis 1000 mg, zweckmässig 100 bis 800 mg und bevorzugt von 200 bis 500 mg angewendet werden können, Weizenkeimoel, z.B. in Mengen von 10 bis 1000 mg, zweckmässig von 100 bis 800 mg und vorzugsweise von 200 bis 500 mg, Lebertran, z.B. in Mengen von 10 bis 1000 mg, zweckmässig von 100 bis 800 mg und vorzugsweise von 200 bis 500 mg.

Gegebenenfalls kann das Mittel weitere Hilfsstoffe, wie Lösungsmittel, Emulgatoren und/oder Stabilisatoren enthalten. Beispiele für Lösungsmittel sind Ethylalkohol, hydriertes Sojaoel, Sojaoel, Erdnussoel (Oleum arachidis) und Wasser. Beispiele für Emulgatoren sind flüssiges Sojalecithin und pulverförmiges Soja-Reinlecithin. Beispiele für Stabilisatoren sind Nipastat und d1-alpha- Tocopherol. Eine besonders bevorzugte Zusammensetzung enthält in ihrer täglich darzureichenden Form 80 - 500 mg Trigonellin oder Trigonellinsäure, vorzugsweise in Form des Extraktes aus den Pflanzen der Unterfamilie Trigonella, 1 - 5 mg Vitamin B 6, 10 -50 mg Nicotinsäure oder Nicotinsäureamid, 1 -4 mg Riboflavin, 0,2 - 1 mg Folsäure, 5 - 25 mg d-Ca-Pantothenat und 1000 -1300 mg Blütenpollen.

Die Verabreichung des Mittels kann auf verschiedene Weise erfolgen. Da die einzelnen Substanzen in Wasser und/oder Aethylalkohol löslich sind, kann beispielsweise eine flüssige Präparation, wie ein Sirup, hergestellt werden. Flüssige Dareichungsformen haben den Nachteil schwieriger dosierbar zu sein. Deshalb ist es vorteilhaft, das erfindungsgemässe Mittel in eine feste Darreichungsform zu bringen. Solche Darreichungsformen sind zum Beispiel Tabletten, Dragees (überzogene Komprimate), Stärkekapseln oder Gelatinekapseln. Die Wirkstoffe in den nach vorliegender Erfindung vorgesehenen Mengen werden je nach Darreichungsform, beispielsweise mit Weizenstärke, Weizenmehl oder Milchzucker zu Stärkekapseln verarbeitet. Eine andere Ausführungsform ist die Gelatinekapsel, wobei die Wirkstoffe in einem Träger, wie einem fetten Oel, einem Glykol, höheren Alkohol, Glykolester, die selbstredend physiologisch unbedenklich sein müssen, aufgenommen und mit Emulgatoren z.B. aus der Reihe der Lecithine, der Gelatinen oder des Caseins und gegebenenfalls einer zugelassenen Menge wenigstens eines Konservierungsmittel, wie PHB-Ester, phenolische Substanzen, Sorbinsäure oder aromatische oder aliphatische Alkohole, und allenfalls weiteren Hilfsstoffen zu derartigen Kapseln verarbeitet werden. Bevorzugt als Kapselmaterial ist hochgereinigte Gelatine, mit und insbesondere ohne Zusätze, wie Farbstoffe.

Vorliegende Erfindung umfasst auch ein kosmetisches Verfahren zur Pflege von Nägeln, Haut und Haaren, zur Bekämpfung fortschreitenden Haarausfalles und zur Anregung neuen Haarwuchses von Humanhaar, mittels Trigonellin als Wirksubstanz, dadurch gekennzeichnet, dass dem menschlichen Körper täglich eine Menge von 80 bis 500 mg Trigonellin zusammen mit 1 bis 5mg Vitamin B6 oral zugeführt werden. Die vorstehend als vorzugsweise bezeichneten Ausführungsformen des Mittels führen bei deren Anwendung zu vorzugsweisen Verfahren.

Die Anwendung des Mittels ist insbesondere menschlichen Lebewesen und zur Bekämpfung des Haarausfalles dabei ganz besonders menschlichen Lebewesen männlichen Geschlechts zugedacht, wobei eine sinngemässe Wirkung bei anderen warmblütigen Lebewesen mit zumindest partiellem Haarkleid denkbar ist.

Ueber die Bekämpfung des Haarausfalles hinaus hat das erfindungsgemässe Mittel eine vorteilhafte Wirkung auf die Qualität und das Aussehen der Haut und der Nägel.

Damit das erfindungsgemässe Mittel seine Wirkung entfalten kann, ist es angezeigt die täglich vorgesehene erfindungsmässige Menge während wenigstens 20 aneinander folgenden Tagen einzunehmen. Entsprechend dem natürlichen Haarwachstum sind erste Erfolge in der Regel nach dieser Zeit feststellbar. Eine obere zeitliche Befristung der Einnahme des Mittels ist nicht zwingend und richtet sich hauptsächlich nach dem gewünschten Erfolg.

Eine Unterstützung der Wirkung durch andere Anwendungsformen des erfindungsgemässen Mittels, beispielsweise durch Lotionen, Tonikum, Shampoos, Cremes oder Salben, die äusserlich an den betroffenen Stellen, demnach beispielsweise auf der Kopfhaut, der Haut allgemein und/oder den Finger- und Zehennägeln angewendet werden, sind im Umfange vorliegender Erfindung.

Lotionen, Tonikum, Shampoos, Cremes und Salben, enthaltend Trigonellin und Vitamin B6 nach vorliegender Erfindung, können auch für sich allein angewendet werden.

Eine bevorzugte Anwendungsform zum äusserlichen Gebrauch, zur Nagel-, Haut- und Haarpflege, zur Bekämpfung fortschreitenden Haarausfalles und zur Anregung neuen Haarwuchses von Humanhaar, ist beispielsweise eine Lotion oder Tonikum mit z.B. Wasser, Ethylalkohol und/oder Propylenglykol als Träger, enthaltend 300 bis 600 mg, vorzugsweise 500 mg, Trigonellin oder Trigonellinsäure, 5 bis 15 mg, vorzugsweise 10 mg, Vitamin B6, 30 bis 50 mg, vorzugsweise 40 mg, Nicotinsäure und/oder Nicotinsäureamid und 60 bis 100 mg, vorzugsweise 80 mg, d-Ca-Pantothenat, jeweils bezogen auf 100 ml Lotion. Weitere wertvolle Stoffe, die einzeln oder zu mehreren in der Lotion enthalten sein können, sind der Reihe von Baumflechtenextrakt, Rosskastanienextrakt, Vitaminkomplex aus den Vitaminen A, E, F und H, kolloidaler Schwefel, Knoblauchextrakt, Phospholipide, Polyoxyethylen-sorbitan- oleat, Ethylnicotinat, einer Mischung von Histidin, Lysin und Arginin im Verhältnis 1:4:12, einem Extrakt aus Brennessel, Huflattich, Schafgarbe, Rosmarin, Salbei, Schachtelhalm, Kleeblüte und Birke, einem wässrigen Extrakt aus Brennesselwurzeln und jungen Brennesselblättern, 2,4-Pyrimidindiamin-6-(1-piperidinyl)-3-oxid, insbesondere in Mengen von 1000 - 3000 mg, vorzugsweise 2000 mg, pro 100 ml Lotion, und schwefelhaltigen Aminosäuren zu entnehmen. Das Knoblauchextrakt enthält beispielsweise die Wirkstoffe Aliin, Aliicin und Methyl-allyl-trisulfid für sich allein oder in einem Gemisch untereinander. Das Knoblauchextrakt wird bevorzugt als alkoholische Lösung von 4 g pro 100 ml Alkohol angewendet.

Andere wertvolle Stoffe, die in einer der äusserlich anzuwendenden Formen des erfindungsgemässen Mittels, beispielsweise in Lotionen, von Interesse sind, können aus der Reine der Stoffe Rizinusoel, Aloe Vera oder Jojoba Oel ausgewählt, werden.

Gegebenenfalls kann die Lotion Tenside, wie Polyoxyethylensorbitan-oleat, Lecithin und Pflanzenduftstoffe enthalten. Ein Shampoo zur Haarpflege und zur Bekämpfung fortschreitenden Haarausfalles und zur Anregung neuen Haarwuchses von Humanhaar enthält 300 -700 mg, bevorzugt 500 mg, Trigonellin oder Trigonellinsäure, 5 - 15 mg, vorzugsweise 10 mg, Vitamin B 6, 30 - 50 mg, vorzugsweise 40 mg, Nicotinsäure und/oder Nicotinsäureamid und 60 - 100 mg, vorzugsweise 80 mg, d-Ca-Pantothenat, pro 100 ml Shampoo Weitere wertvolle Stoffe die in derartigen Shampoos enthalten sein können sind beispielsweise aus der Reihe von Baumflechtenextrakt, Rosskastanienextrakt, Vitaminkomplex aus den Vitaminen A, E, F und H, Phospholipide, Polyoxyethylen-sorbitan-oleat, Ethylnicotinat, einer Mischung von Histidin, Lysin und Arginin im Verhältnis 1:4:12, einem Extrakt aus Brennessel, Huflattich, Schafgarbe, Rosmarin, Salbei, Schachtelhalm, Kleeblüte und Birke, einem wässrigen Extrakt aus Brennesselwurzeln und jungen Brennesselblättern und von Lecithin auszuwählen. Die obengenannten Bestandteile und Wirkstoffe können in ihrer auf chemische oder biochemische Weise gewonnen Form oder, soweit auf pflanzlicher Basis beruhend, in ihren natürlich vorkommenden Formen eingesetzt werden. Produkte auf natürlicher Basis oder aus Naturstoffen gewonnen, werden bevorzugt.

Finger- und Zehennägel können auch mit kosmetischen Formulierungen im Sinne von Nagellack, der das Mittel enthält, behandelt werden.

## Patentansprüche

1. Mittel zur Nagel-, Haut- und Haarpflege, zur Bekämpfung fortschreitenden Haarausfalles und zur Anregung neuen Haarwuchses von Humanhaar, enthaltend in der täglich darzureichenden Form
a) 80 bis 500 mg Trigonellin und
b) 1 bis 5 mg Vitamin B6.

2. Mittel gemäss Anspruch 1, enthaltend
a) 100 bis 400 mg Trigonellin und
b) 2 bis 4 mg Vitamin B6.

3. Mittel gemäss Anspruch 1, enthaltend 10 bis 50 mg Nicotinsäure und/oder Nicotinsäureamid.

4. Mittel gemäss Anspruch 1, enthaltend 1 bis 4 mg Riboflavin.

5. Mittel gemäss Anspruch 1, enthaltend 0,2 bis 1mg Folsäure.

6. Mittel gemäss Anspruch 1, enthaltend 5 bis 25 mg d-Calcium-Pantothenat.

7. Mittel gemäss Anspruch 1, enthaltend die Aminosäuren Cystein und/oder Lysin.

8. Verwendung des Mittels nach einem der ansprüche 1 bis 7 als Zusatzstoff für Shampoos oder Haarwasser.

9. Verwendung des Mittel nach einem der ansprüche 1 bis 7 zur Pflege von Nägeln und Haut.

10. Kosmetisches Verfahren zur Bekämpfung fortschreitenden Haarausfalles und zur Anregung neuen Haarwuchses von Humanhaar, dadurch gekennzeichnet, daß dem menschlichen Körper täglich eine Menge von 80 bis 500 mg Trigonellin zusammen mit 1 bi 5 mg Vitamin B6 oral zugeführt werden.

11. Kosmetisches Verfahren zur Pflege von Nägeln und Haut dadurch gekennzeichnet, daß dem menschlicheb Körper täglich eine Menge von 80 bis 500 mg Trigonellin zusammen mit 1 bis 5 mg Vitamin B6 oral zugeführt werden.

12. Mittel gemäss Anspruch 1, enthaltend in der täglich darzureichenden Form 80 - 500 mg Trigonellin oder Trigonellinsäure, vorzugsweise in Form des Extraktes aus den Pflanzen der Unterfamilie Trigonella, 1 - 5 mg Vitamin B 6, 10 - 50 mg Nicotinsäure oder Nicotinsäureamid, 1 -4 mg Riboflavin, 0,2 - 1 mg Folsäure, 5 - 25 mg d-Ca-Pantothenat und 1000 -1300 mg Blütenpollen.

13. Lotion oder Tonikum zur Nagel-, Haut- und Haarpflege, zur Bekämpfung fortschreitenden Haarausfalles und zur Anregung neuen Haarwuchses von Humanhaar, enthaltend Wasser, Ethylalkohol und/oder Propylenglykol als Träger und 300 bis 700 mg, vorzugsweise 500 mg, Trigonellin oder Trigonellinsäure, 5 bis 15 vorzugsweise 10 mg, Vitamin B6, 30 bis 50 mg, vorzugsweise 40 mg, Nicotinsäure und/oder Nicotinsäureamid und 60 bis 100 mg, vorzugsweise 80 mg, d-Ca-Pantothenat, jeweils bezogen auf 100 ml Lotion.

14. Shampoo zur Haarpflege und zur Bekämpfung fortschreitenden Haarausfalles und zur Anregung neuen Haarwuchses von Humanhaar, enthaltend 300 -700 mg, bevorzugt 500 mg, Trigonellin oder Trigonellinsäure, 5 - 15 mg, vorzugsweise 10 mg, Vitamin B 6, 30 - 50 mg, vorzügsweise 40 mg, Nicotinsäure und/oder Nicotinsäureamid und 60 - 100 mg, vorzugsweise 80 mg, d-Ca-Pantothenat, pro 100 ml Shampoo.

## Claims

1. Agent for nail, skin and hair care, for combating advancing hair loss and for stimulating new hair growth of human hair containing
a) 80 to 500 mg of trigonelline and
b) 1 to 5 mg of vitamin B6 in the form to be administered daily.

2. Agent according to claim 1 containing
a) 100 to 400 mg of trigonelline and
b) 2 to 4 mg of vitamin B6.

3. Agent according to claim 1 containing 10 to 50 mg of nicotinic acid and/or nicotinic acid amide.

4. Agent according to claim 1 containing 1 to 4 mg of riboflavin.

5. Agent according to claim 1 containing 0.2 to 1 mg of folic acid.

6. Agent according to claim 1 containing 5 to 25 mg of d-calcium pantothenate.

7. Agent according to claim 1 containing the amino acids cysteine and/or lysine.

8. Use of the agent according to one of claims 1 to 7 as additive for shampoos or hair lotions.

9. Use of the agent according to one of claims 1 to 7 for care of nails and skin.

10. Cosmetic process for combating advancing hair loss and for stimulating new hair growth of human hair, characterised in that a quantity of 80 to 500 mg of trigonelline together with 1 to 5 mg of vitamin B6 is supplied orally to the human body daily.

11. Cosmetic process for the care of nails and skin, characterised in that a quantity of 80 to 500 mg of trigonelline together with 1 to 5 mg of vitamin B6 is supplied orally to the human body daily.

12. Agent according to claim 1 containing 80 - 500 mg of trigonelline or trigonellinic acid, preferably in the form of the extract from the plants of the subfamily Trigonella, 1 - 5 mg of vitamin B6, 10 - 50 mg of nicotinic acid or nicotinic acid amide, 1 - 4 mg of riboflavin, 0.2 - 1 mg of folic acid, 5 - 25 mg of d-Ca pantothenate and 1,000 - 1,300 mg of flower pollen, in the form to be administered daily.

13. Lotion or tonic for nail, skin and hair care for combating advancing hair loss and for stimulating new hair growth of human hair, containing water, ethyl alcohol and/or propylene glycol as excipient and 300 to 700 mg, preferably 500 mg, of trigonelline or trigonellinic acid, 5 to 15, preferably 10 mg, of vitamin B6, 30 to 50 mg, preferably 40 mg, of nicotinic acid and/or nicotinic acid amide and 60 to 100 mg, preferably 80 mg, of d-Ca pantothenate, based in each case on 100 ml of lotion.

14. Shampoo for hair care and for combating advancing hair loss and for stimulating new hair growth of human hair, containing 300 to 700 mg, preferably 500 mg, of trigonelline or trigonellinic acid, 5 to 15 mg, preferably 10 mg, of vitamin B6, 30 to 50 mg, preferably 40 mg, of nicotinic acid and/or nicotinic acid amide and 60 to 100 mg, preferably 80 mg, of d-Ca pantothenate, per 100 ml of shampoo.

## Revendications

1. Produit utilisé pour soigner les ongles, la peau et les cheveux, pour combattre la chute progressive des cheveux et pour stimuler la repousse des cheveux humains, comprenant, dans la forme à présenter quotidiennement :
a) 80 à 500 mg de trigonelline et
b) 1 à 5 mg de vitamine B6.

2. Produit selon la revendication 1, comprenant :
a) 100 à 400 mg de trigonelline et
b) 2 à 4 mg de vitamine B6.

3. Produit selon la revendication 1, comprenant 10 mg à 50 mg d'acide nicotinique et/ou d'amide d'acide nicotinique.

4. Produit selon la revendication 1, comprenant 1 mg à 4 mg de riboflavine.

5. Produit selon la revendication 1, comprenant 0,2 mg à 1 mg d'acide folique.

6. Produit selon la revendication 1, comprenant 5 mg à 25 mg de pantothénate-d-calcium.

7. Produit selon la revendication 1, comprenant les acides aminés cystéine et/ou lysine.

8. Utilisation du produit selon l'une quelconque des revendications 1 à 7, comme additif pour shampoings ou lotions pour les cheveux.

9. Utilisation du produit selon l'une quelconque des revendications 1 à 7, pour soigner les ongles et la peau.

10. Procédé cosmétique pour combattre la chute progressive des cheveux et stimuler la repousse des cheveux humains, caractérisé en ce qu'une quantité de 80 à 500 mg de trigonelline combinée avec 1 à 5 mg de vitamine B6 est administrée au corps humain par voie orale.

11. Procédé cosmétique pour soigner les ongles et la peau, caractérisé en ce qu'une quantité de 80 à 500 mg de trigonelline combinée avec 1 à 5 mg de vitamine B6 est administrée au corps humain par voie orale.

12. Produit selon la revendication 1, comprenant, dans la forme à présenter quotidiennement, 80 à 500 mg de trigonelline ou d'acide de trigonelline, de préférence sous la forme d'un extrait de plantes de la sous-famille trigonella, 1 à 5 mg de vitamine B6, 10 à 50 mg d'acide nicotinique ou d'amide d'acide nicotinique, 1 à 4 mg de riboflavine, 0,2 à 1 mg d'acide folique, 5 à 25 mg de pantothénate-d-Ca et 1000 à 1300 mg de pollen.

13. Lotion ou tonique pour soigner les ongles, la peau et les cheveux, pour combattre la chute progressive des cheveux et pour stimuler la repousse de nouveaux cheveux humains, comprenant, pour 100 ml de lotion, de l'eau, de l'alcool éthylique et/ou du propylèneglycol comme véhicule, ainsi que 300 à 700 mg, de préférence 500 mg, de trigonelline ou d'acide de trigonelline, 5 à 15 mg, de préférence 10 mg, de vitamine B6, 30 à 50 mg, de préféfence 40 mg, d'acide nicotinique et/ou d'amide d'acide nicotinique, ainsi que 60 à 100 mg, de préférence 80 mg, de pantothénate-d-Ca.

14. Shampoing pour soigner les cheveux et combattre la chute progressive des cheveux, ainsi que pour stimuler la repousse de nouveaux cheveux humains, comprenant, pour 100 ml de shampoing, 300 à 700 mg, de préférence 500 mg, de trigonelline ou d'acide de trigonelline, 5 à 15 mg, de préférence 10 mg, de vitamine B6, 30 à 50 mg, de préférence 40 mg, d'acide nicotinique et/ou d'amide d'acide nicotinique, ainsi que 60 à 100 mg, de préférence 80 mg, de pantothénate-d-Ca.
